# EUROPEAN PATENT SPECIFICATION

(11) **EP 1 788 878 B1**
(45) Date of publication and mention of the grant of the patent: **15.10.2008**
(21) Application number: 05777114.9
(22) Date of filing: 30.08.2005
(51) Int. Cl.: A01N 49/00, A01N 43/90, A01N 43/16

(54) **ANTIBACTERIAL COMPOSITION**
ANTIBAKTERIELLE ZUSAMMENSETZUNG
COMPOSITION ANTIBACTERIENNE

(30) Priority: 09.09.2004 JP 2004262903
(43) Date of publication of application: 30.05.2007
(73) Proprietor: Takasago International Corporation, Tokyo-to 144-8721 (JP)
(72) Inventor: TAKEUCHI, Ryo, TAKASAGO INTERNATIONAL CORPORATION, Hiratsuka-shi, Kanagawa 2540073 (JP); HIRAMOTO, Tadahiro, TAKASAGO INTERNATIONAL CORP., Hiratska-shi, Kanagawa 2540073 (JP)
(74) Representative: Gillard, Richard Edward
(86) International application number: PCT/JP2005/016181
(87) International publication number: WO 2006/028025

(56) References cited:
- DATABASE CA [Online] CHEMICAL ABSTRACTS SERVICE, COLUMBUS, OHIO, US; IMOHARA, ASANO ET AL: "Coumarin derivatives isolation from lemon as antioxidants" XP002363608 retrieved from STN Database accession no. 1995:777823 & JP 02 864436 B2 (TAKASAGO PERFUMERY CO LTD, JAPAN) 3 March 1999 (1999-03-03) cited in the application

## Description

### TECHNICAL FIELD

The present invention relates to an antibacterial composition comprising 9-(3',7'-dimethyl-2',6'-octadienyl)-4-hydroxy-7H-furo[3,2-γ][1]benzopyran-7-one and 8-(3',7'-dimethyl-2',6'-octadienyl)-5-hydroxy-7-methoxy-2H-1-benzopyran-2-one. This antibacterial composition has an improved antibacterial effect for a wide range of bacterial species, is safe for human and the environment, and is tasteless and odorless by itself that it does not change the smell or the taste of the food or the like being added therewith, thus finds itself in many applications.

### BACKGROUND ART

Traditionally, antibacterial agents and antiseptic agents have been used in various fields such as food products, cosmetic products, oral products, pharmaceuticals medicated cosmetics and daily goods to prevent deterioration in quality or flavor associated with the growth of harmful microorganisms or to deal with unpleasant odors associated with the growth of microorganisms.

For example, food products are added with antiseptic agents for longer shelf life and food poisoning prevention. Oral products are added with antibacterial agents to prevent and control the growth of cariogenic bacteria and periodontal bacteria that cause tooth cavities and periodontal disease, respectively. Moreover, cosmetic products are added with antibacterial agents to control the growth of armpit odor bacteria, dandruff-causative bacteria and acne bacteria that cause armpit odor, dandruff and acnes, respectively.

However, since conventionally used antibacterial agents and antiseptic agents do not have adequate antibacterial activity or adequately wide antibacterial spectrum, little of them show satisfactory effects. In addition, even agents having antibacterial activities and wide antibacterial spectra are not always satisfactory in terms of safety that they could be great pressure on human and the environment.

For example, paraben conventionally and frequently used as an antiseptic agent in cosmetic products has a narrow antibacterial spectrum and a weak activity and thus has not always been a satisfactory antibacterial agent. Triclosan known as an antibacterial substance with a stronger antibacterial activity and a wider antibacterial spectrum harbors a chlorine atom in its molecule and thus limited of its use in products for the concern about the effects on the environment and human body.

In addition, bacteria such as heat-resistant acidophilic bacteria (Genus *Alicyclobacillus*) that grow flavoring high temperature (40-70°C) and acidic (pH 2-6) conditions are known. In normal sterilization conditions for soft drink (86-96°C, 2 min.), these bacteria proliferate to give unpleasant chemical smell, diminish flavor or cause turbidity, thereby significantly reducing the commercial value of the product. In order to avoid this, an antibacterial effect of sucrose fatty acid ester against heat-resistant acidophilic bacteria is acknowledged and thus proposed as an additive in low-acid beverages such as a coffee milk beverage. The ester, however, is poorly dispersed in an acidic region and likely to crystallize, causing turbidity and sediment in an acidic beverage and thus pointed out of its disadvantage of reducing the commercial value of the product.

Moreover, due to the recent increase of sanitary and safety concerns by the consumers, highly safe antibacterial agents with higher antibacterial actions and wider antibacterial spectra have been desired especially for products that directly touches human body such as food products, oral products and cosmetic products.

Database CA [Online] Chemical Abstracts Service, Columbus, Ohio, US; Imohara *et al*., Database accession no. 1995: 777823, corresponding to JP 02 864 436 B2 discloses coumarin derivatives of formula I: 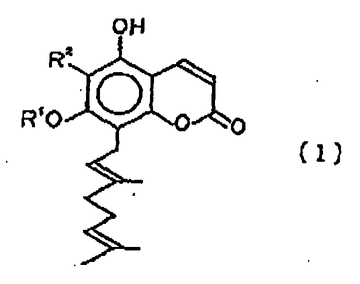 Wherein R¹=Me and R²=H **or** R¹ and R² together are vinylene, and the use of these compounds as antioxidants.

### DISCLOSURE OF THE INVENTION

Thus, the object of the present invention is to provide an antibacterial composition which is unharmful and safe for human and the environment and which is not a chloride compound, with a stronger antibacterial activity and a wider antibacterial spectrum compared to conventional antibacterial agents.

In order to obtain an antibacterial composition with an improved and safe antibacterial action, the present inventors have searched for a substance having a high antibacterial action and found that a combination of 9-(3',7'-dimethyl-2',6'-octadienyl)-4-hydroxy-7H-furo[3,2-γ][1]benzopyran-7-one and 8-(3',7'-dimethyl-2',6'-octadienyl)-5-hydroxy-7-methoxy-2H-1-benzopyran-2-oneknown as antioxidants exerts an improved antibacterial effect against a wide range of bacterial species.

Thus, the present invention solves the above-described problems by providing an 9-(3',7'-dimethyl-2',6'-octadienyl)-4-hydroxy-7H-furo[3,2-γ][1]benzopyran-7-one and 8-(3',7'-dimethyl-2',6'-octadienyl)-5-hydroxy-7-methoxy-2H-1-benzopyran-2-one The present invention also provides food products, cosmetic products, oral products and pharmaceutical products that are added with this novel antibacterial composition.

According to the present invention, an antibacterial composition with an improved antibacterial effect, with an antibacterial action against a wider range of bacteria than a conventional antibacterial agent butylparaben and with higher safety, as well as a food product, a pharmaceutical product, an oral product, a cosmetic product and the like comprising the antibacterial composition of the invention are provided.

### BEST MODE FOR CARRYING OUT THE INVENTION

Hereinafter, the present invention will be described in more detail.

"Antibacterial composition" of the present invention refers to an improved agent having the property as antiseptic and disinfectant agents, comprising, as an active element, a mixture of (a) 9-(3',7'-dimethyl-2',6'-octadienyl)-4-hydroxy-7H-furo[3,2-γ][1]benzopyran-7-one (Formula (1) below) and (b) 8-(3',7'-dimethyl-2',6'-octadienyl)-5-hydroxy-7-methoxy-2H-1-benzopyran-2-one (Formula (2) below).

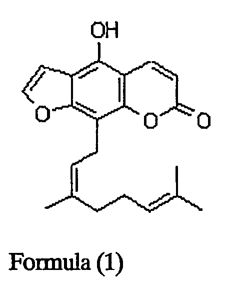

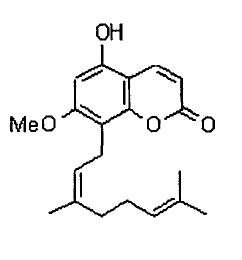

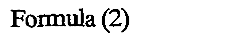

Each compound can be obtained according to the method described in Japanese Patent No. 2864436. Specifically, lemon oil is first subjected to reduced-pressure distillation. To the residue, methanol in 10 times or more volume (preferably, 10-20 times volume) of the residue is added, agitated with heating for dispersion and left in a freezer overnight. Thereafter, the resultant is separated into a methanol solution and methanol-insoluble wax-like sediment, which are filtrated with a filter aid such as celite. The solution is subjected to vacuum concentration to obtain an oil-like substance. The oil-like substance is then dissolved in an organic solvent and separated into strongly-acidic part, neutral part and weakly-acidic part. Appropriate organic solvents are esters such as methyl acetate, ethyl acetate and propyl acetate, and ethers such as diethyl ether and dipropyl ether, preferably ethyl acetate. First, the organic solvent phase is extracted with a weakly-alkaline aqueous solution. And the extract is neutralized with acid and extracted with the organic solvent to obtain a strongly-acidic fraction (Fraction SA). Next, the organic solvent phase is extracted with a strongly-alkaline aqueous solution. And the extract is neutralized with acid and extracted with the organic solvent to obtain a weakly acidic fraction (Fraction WA). The organic solvent phase separated from the strongly-alkaline aqueous solution phase is washed with water and concentrated to obtain a neutral part (Fraction N). Then, each of the compounds is obtained by being isolated from the weakly-acidic fraction having strong antioxidant ability by column chromatography.

The antibacterial composition according to the present invention exerts its action as an antibacterial composition only after mixing the above compounds to enhance the antibacterial activity to a practical level. The proportion of (a) and (b) may vary depending on the final product, but an antibacterial composition with a weight ratio (a):(b) of 1:30 to 30:1 is desirable. In order to further improve the synergic effect of the antibacterial activity of the mixture of Component (a) and Component (b), an antibacterial composition with a weight ratio (a):(b) of 1:10-10:1 is more desirable and an antibacterial composition with a weight ratio (a):(b) of 1:8 to 8:1 is particularly desirable.

The thus-obtained antibacterial composition of the present invention exerts improved antibacterial effects against various bacteria. Examples of these bacteria include cariogenic bacteria, periodontal bacteria, acne bacteria, abscess bacteria, armpit odor bacteria, dandruff-causative bacteria, resident skin bacteria, putrefactive bacteria, food poisoning bacteria, gastric ulcer-causative bacteria, staphyrococcus aureus and heat-resistant acidophilic bacteria, preferably cariogenic bacteria, periodontal bacteria, acne bacteria, abscess bacteria, putrefactive bacteria, staphyrococcus aureus and heat-resistant acidophilic bacteria.

Specific examples of these bacteria are as follows.
Cariogenic bacteria;*Actinomyces naeslundii, Actinomyces viscosus, Streptococcus mutans.*
Periodontal bacteria; *Fusobacterium nucleatum, Prevotella intermedia, Porphyromonas gingivalis*.
Acne bacteria; *Propionibacterium acnes.*
Abscess bacteria; *Bacteroides fragilis.*
Armpit odor bacteria; *Corynebacterium xerosis.*
Dandruff-causative bacteria; *Malassezia furfur.*
Resident skin bacteria; *Staphyrococcus epidermidis, Corynebacterium minutissimum.*
Putrefactive bacteria; *Bacillus subtilis.*
Food poisoning bacteria; *Vibrio parahaemoliticus, Campylobactor jejuni*.
Gastric ulcer-causative bacteria; *Helicobacter pylori.*
Staphyrococcus aureus; *Staphyrococcus aureus,*
Heat-resistant acidophilic bacteria; *Alicyclobacillus acidocaldarius* (NCIMB), *Alicyclobacillus acidoterrestris* (NCIMB).

The thus-obtained mixture of the above compounds can directly be added as an antibacterial composition to food products and the like. The mixture of the above compounds can be dissolved or dispersed in a suitable liquid carrier (e.g., ethanol, aqueous ethanol, benzyl alcohol, medium-chain triglyceride (MCT), etc.), or mixed with or adsorbed to a suitable powder carrier (e.g., polysaccharides, processed starch, active carbon, silica gel, etc.) to be used as an antibacterial composition of the invention. In some cases, an emulsifying agent, a dispersant, a suspending agent, a spreading agent, a penetrant, a wetting agent or a stabilizer can be added to formulate an emulsion, a hydrating agent, powder or a tablet to be used as an antibacterial composition for food products, cosmetic products, oral products or pharmaceuticals.

Furthermore, the antibacterial composition of the invention may be mixed with a blending agent or a combination of two or more blending agents such as a filler, an antioxidant, a pigment, known antiseptic and antibacterial agent, a deodorant substance, a surfactant, a flavoring ingredient, a stabilizer, an absorbent (calcium chloride, highly water absorbent polymer, etc.) and an excipient (lactose, etc.) to prepare a characteristic antibacterial composition.

For example, when the antibacterial composition of the invention is incorporated into a cosmetic product, a toiletry product, an oral product or the like, a flavoring ingredient is preferably added to the antibacterial composition so that it can create an image of cleanliness. When the antibacterial composition of the invention is incorporated into a food product, a flavoring ingredient is also preferably added to the antibacterial composition so that it can complement the flavoring ingredient which was lost from the food product for the various reasons. A surfactant is preferably added to the antibacterial composition of the invention so that when the antibacterial composition is added to the product, the surfactant enables the antibacterial composition to dissolve or disperse in the product effectively. A deodorant substance is preferably added to the antibacterial composition of the invention so that it can remove unpleasant odor as well as restrain the proliferation of the bacteria which cause such odor. A filler is preferably added to the antibacterial composition of the invention so that the antibacterial composition can be used in powdery form as well as liquid form. A known antiseptic and/or antibacterial agent is preferably added to the antibacterial composition of the invention in order to exert antibacterial effect against the broadest possible range of bacteria.

The amount of the blending agent is not limited as long as the primary object is achieved.

Examples of fillers include saccharides, polysaccharides, processed starch, casein, gelatin, carboxymethyl cellulose (hereinafter, referred to as "CMC") and lecithin.

Examples of antioxidants include butylhydroxytoluene, butylhydroxyanisole, citric acid, biofulvic acid, glutathione, selenium, lycopene, vitamin A, vitamin E, vitamin C as well as pyrrolopyrrole derivatives, free radical scavengers obtained from various plant extracts, superoxide dismutase, and enzymes with antioxidant property such as glutathione peroxidase.

As pigments, dye, lake, synthetic pigment such as organic color (tarcolor) and natural pigments are known, and examples thereof specifically include hibiscus pigment, huckleberry pigment, plum pigment, laver pigment, dewberry pigment, grape juice pigment, blackberry pigment, blueberry pigment, mulberry pigment, morello cherry pigment, red currant pigment, loganberry pigment, powdered paprika, malt extract, rutin, flavonoid, red cabbage pigment, red radish pigment, Adzuki bean pigment, turmeric pigment, olive tea, cowberry pigment, powdered chlorella, saffron pigment, perilla pigment, strawberry pigment, chicory pigment, pecan nut pigment, monascus pigment, safflower pigment, purple sweet potato pigment, lac pigment, spirulina pigment, onion pigment, tamarind pigment, capsicum pigment, gardenia pigment, lithospermi radix pigment, rose wood pigment, krill pigment, orange pigment, carrot carotene, caramel, titanium dioxide, sodium iron chlorophyllin, riboflavin, potassium norbixin, sodium norbixin, amaranth, erythrosin, new coccine, phloxine B, rose bengal, acid red, tartrazine, sunset yellow, fast green, brilliant blue, indigo carmine, lake red C, lithol red, rhodamine, phloxine, indigo, ponceau, orange I, Sudan blue, mica, talc, calcium carbonate, kaolin, silicic acid anhydride, aluminum oxide, Bengara, ferric oxide, ultramarine, carbon black, zinc oxide, isinglass, bismuth oxychloride, boron nitride, photochromic color, particulate complex powder (hybrid fine powder) and synthetic mica.

Examples of known antiseptic agents and antibacterial agents include benzoic acid, sodium benzoate, isopropyl paraoxybenzoate, isobutyl paraoxybenzoate, ethyl paraoxybenzoate, methyl paraoxybenzoate, butyl paraoxybenzoate, propyl paraoxybenzoate, sodium sulfite, sodium hyposulfite, potassium pyrosulfite, sorbic acid, potassium sorbate, sodium dehydroacetate, thujaplicin, udo (*Aralia cordata*) extract, storax extract, artemisia capillaris extract, oolong tea extract, soft roe protein extract, enzyme-degraded job's tear extract, tea catechins, apple polyphenol, pectin-degrading substance, chitosan, lysozyme and e-polylysine.

Examples of known deodorants include deodorants with desulfurization action (e.g., iron sulfate such as ferrous sulfate or iron hydrochloride), deodorants with chemical action (e.g., acidic agent, alkaline agent, oxidant or reductant), deodorants with addition/condensation action (addition agent: (meta)acrylic acid ester, ester maleate, etc.; condensation agent: glyoxysal, etc.), deodorants with ion exchange action (e.g., amphoteric agent, cation agent and anion agent for ion exchange resin), deodorants with drug impregnated adsorption action (alkaline or acidic impregnated active carbon and mixture of active carbon and chemical reactant), deodorants with adsorption action (e.g., neutral active carbon, fibrous carbon adsorbent, zeolite and porous adsorbents such as activated clay), deodorants with absorbing action (e.g., organic solvents such as alcohol and hexane, water and surfactant), deodorants with enzyme action (e.g., digestive enzyme, good oral bacteria LS-1 lactic acid bacteria, yeast and soil bacteria), deodorants with antiseptic/sterilizing action (e.g., chloramine T, parabens and phenols), polyphenol deodorants (e.g., persimmon polyphenol, tea catechin, rosemary extract, oolong tea extract, tansy extract, *Quercus salicina* leaf extract and rice bran/soybean roasted extract), cyclodextrin, champignon extract, Rooibos extract, sodium iron chlorophin, active carbon and zeolite.

Preferably, surfactant is one or a combination of two or more surfactants of nonionic type, specifically polyoxyethylenealkylether and fatty acid alkylol amide, or acylglutamic acid. Examples of polyoxyethylenealkylether include polyoxyethylenestearyl and polyoxyethylene hydrogenated castor oil. An example of fatty acid alkylol amide includes coconut oil fatty acid diethanolamide. Examples of acylglutamic acid type include glutamic acid ester of saturated and unsaturated fatty acid with a carbon number of 12-18 or a mixture thereof such as coconut oil fatty acid, hydrogenated coconut oil fatty acid, palm oil fatty acid, hydrogenated palm oil fatty acid, tallow fatty acid and hydrogenated tallow fatty acid, and specifically include
N-coconut oil fatty acid acyl-L-glutamic acid triethanolamine, lauroyl-L-glutamic acid triethanolamine, N-coconut oil fatty acid acyl-sodium L-glutamate, N-lauroyl-sodium L-glutamate, N-myristoyl-sodium L-glutamate, N-coconut oil fatty acid/hydrogenated tallow fatty acid acyl-sodium L-glutamate and N-coconut oil fatty acid acyl-potassium L-glutamate.

Furthermore, a flavoring ingredient (flavor or fragrance) may be blended with the antibacterial composition. As a result, aroma can be added to further improve usability.

The blending quantity of a flavoring ingredient varies on the application and usage of the antibacterial composition, but usually 0.001-50% by weight of the antibacterial composition is preferable.

Examples of flavors used with the present invention include synthetic flavoring ingredients such as esters, alcohols, aldehydes, ketones, acetals, phenols, ethers, lactones, furans, hydrocarbons and acids as well as natural flavoring ingredients.

Examples of fragrances used with the present invention include hydrocarbons, alcohols, phenols, aldehydes and/or acetals, ketones and/or ketals, ethers, synthetic musks, acids, lactones, esters, halogen-containing compounds as well as natural flavoring ingredient.

Other than the above flavors and fragrances, flavoring ingredients described in "Field survey of food flavoring ingredient compounds used in Japan" (2000, Health Science Study Report; Japan Flavor and Fragrance Materials Association, issued March 2001); "Synthetic flavoring ingredient chemicals and product knowledge" (Genichi Indo, issued March 6, 1996, Chemical Daily Co., Ltd.); and "Perfume and Flavor Chemicals (Aroma Chemicals) 1, 2" (Steffen Arctender (1969)) may be used.

These flavors and fragrances may be used alone or in combination of two or more.

Commercially available flavors and fragrances may also be used. Each of them may be synthetic or derived from a natural source such as a plant. Essential oils, resinoid, balsam, absolute, concrete and tincture may be prepared by a known method.

The antibacterial composition of the present invention may be added to and blended with, for example, a food product, a cosmetic product (a fragrance product, a skin-care cosmetic, a hair-care cosmetic, a toiletry product, a bath additive, a body-care product, a detergent, a softener, an aromatic deodorant), an oral product and a pharmaceutical product but not limited thereto. Preferably, the antibacterial composition of the present invention may be added to and blended with a food product and an oral product.

Examples of the above food products include beverages such as fruit beverage, non-fruit beverage, tea beverage, lactic acid beverage and powder beverage, frozen desserts such as ice cream, sherbet and ice dessert, desserts such as pudding, jelly, Bavarian cream and yogurt, confectioneries such as gum and candy and fish-paste products.

Examples of the above fragrance product include perfume, eau de toilette, eau de cologne and shower cologne.

Examples of the above skin-care cosmetics include skin cream, cleansing cream, skin lotion, after-shaving lotion, foundation, lipstick and talcum powder.

Examples of the above hair-care cosmetics include hair-care products such as shampoo, rinse, conditioner, rinse-in-shampoo and treatment agent, hair styling agents such as pomade, hair tonic, hair liquid and hair gel, hair-growth agent, hair dye and cold wave lotion.

Examples of the above toiletry products include cosmetic soap, bath soap and transparent soap.

Examples of the above detergents include a powdered fabric detergent, liquid fabric detergent, a softener, a kitchen detergent, a toilet detergent, a bathroom detergent, a glass cleaner and a mildew remover.

Examples of the above bath additives include bath powder, bath cake, bubble bath cake, bath oil and bubble bath.

Examples of the above aromatic deodorants include a gel aromatic deodorant, a mist aromatic deodorant and an impregnated aerosol aromatic deodorant.

Examples of the above pharmaceuticals include a tablet, a liquid drug, a capsule-type drug and a granular drug.

Examples of the above oral products include a mouth wash, a tooth paste, an oral care gum and an oral care candy.

The amount of additives added to or blended with the above-described antibacterial composition greatly varies depending on the subject ("the subject" may be the final product or the raw material of the final product) to be added and bacteria to be aimed, but usually, an amount of 0.0000001-50% by weight of the subject is preferable. When the amount is 0.0000001% by weight or less, the antibacterial activity ability will be inadequate, and when 50% by weight or more, the antibacterial activity ability is adequate but disadvantageous in economic terms. Most preferably, the amount is 0.0001-10% by weight of the subject.

The antibacterial composition of the invention shows strong antibacterial effects against, for example, cariogenic bacteria, periodontal bacteria, acne bacteria, armpit odor bacteria, dandruff-causative bacteria, resident skin bacteria, abscess bacteria and food poisoning bacteria.

Food poisoning bacteria lead to rottenness and deterioration of food products, thereby greatly reducing the commercial value of the product. Moreover, as the bacteria proliferate, toxic is produced in the product, having serious adverse effect on those who eat the product. Thus, by adding the antibacterial composition of the invention to a food product, longer shelf-life and prevention of food poisoning can be achieved.

Cariogenic bacteria cause tooth caries. Periodontal bacteria cause periodontal disease. In addition, these bacteria orally proliferate and become a source of bad breath. Thus, the antibacterial composition of the invention can be contained in oral products to prevent tooth cavities, periodontal disease and bad breath.

The antibacterial composition of the invention can be contained in various cosmetic products and the like to control proliferation of dandruff-causative bacteria, acne bacteria, armpit odor bacteria and abscess bacteria, expecting its effects in deodorant, dandruff prevention and acnes prevention.

### EXAMPLES

Hereinafter, the present invention will be described in more detail with reference to Examples and comparative examples, which do not limit the present invention.

### [EXAMPLE:1]

### Preparation of antibacterial composition (water-soluble antibacterial composition)

9-(3',7'-dimethyl-2',6'-octadienyl)-4-hydroxy-7H-furo[3,2-γ][1]benzopyran-7-one (a) and 8-(3',7'-dimethyl-2',6'-octadienyl)-5-hydroxy-7-methoxy-2H-1-benzopyran-2-one (b) prepared according to the method described in Japanese Patent No. 2864436, respectively, are mixed at the following weight ratios to prepare antibacterial compositions. Each antibacterial composition is made into a 0.2% ethanol solution before being used in the Examples.

| | |
|---|---|
| Antibacterial composition (A) | (a):(b)=1:8 |
| Antibacterial composition (B) | (a):(b)=1:1 |
| Antibacterial composition (C) | (a):(b)=8:1 |
| Antibacterial composition (D) | (a):(b)=1:30 |
| Antibacterial composition (E) | (a):(b)=30:1 |
| Antibacterial composition (F) | (a):(b)=0:1 |
| Anti-bacterial composition (G) | (a):(b)=1:0 |

### [EXAMPLE: 2]

### Preparation of antibacterial composition (oil-soluble antibacterial composition)

9-(3',7'-dimethyl-2',6'-octadienyl)-4-hydroxy-7H-furo[3,2-γ][1]benzopyran-7-one (a) and 8-(3',7-dimethyl-2',6'-octadienyl)-5-hydroxy-7-methoxy-2H -1-benzopyran-2-one (b) prepared according to the method described in Japanese Patent No. 2864436, respectively, are mixed at the following weight ratios to prepare antibacterial compositions. Each antibacterial composition is made into a 0.2% medium-chain triglyceride (MCT) solution before being used in the Examples.

| | |
|---|---|
| Antibacterial composition (H) | (a):(b)=1:8 |
| Antibacterial composition (I) | (a):(b)=8:1 |
| Antibacterial composition (J) | (a):(b)=1:1 |

### [EXAMPLE: 3]

### Determination of Minimum Inhibitory Concentration (MIC)

Minimum inhibitory concentration (MIC) was determined by Agar Dilution Test (ADT).

Each antibacterial composition obtained in Example 1 was dissolved in ethanol or water (according to solubility of each antibacterial composition) to carry out two-fold serial dilution. A hundred *µ*l of the resultant was added to a 10ml sterilized agar medium (skin-related bacteria and standard bacteria in Mueller-Hinton Medium (Difco), yeast and filamentous bacteria in Sabroud Medium(Difco) and anaerobic bacteria in Trypticase Soy agar(BBL)), well agitated, transferred to a 9 cmf petri dish and immobilized at room temperature. To this petri dish, a 27-spot microplanter MIT-P from Sakuma Seisakusho, LTD. was used to spot 1 x 10⁵ cfu (colony forming unit) of skin-related bacteria and standard bacteria, and the resultants were cultured at 37°C for 18 hours. Anaerobic bacteria are cultured at 37°C for 72 hours under anaerobic conditions (using BBL gas pack anaerobic system). After completion of cultivation, growth of each bacterium was compared with that in a petri dish with alcohol alone (blank). A concentration of a sample without a bacterium growth was determined as the minimum inhibitory concentration (MIC).

As comparative substances, butylparaben (BPHB) and triclosan were determined as well. Results are as follows.

**Table 1**

| | Triclosan | Butylparaben | Antibacterial composition (A) | Antibacterial composition (F) | Antibacterial composition (G) |
|---|---|---|---|---|---|
| Se-1 | 1.6 | 200 | 12.5 | 200 | 200 |
| Se-2 | 1.6 | 200 | 12.5 | 200 | 200 |
| Cx- | 23.1 | 50 | 6.3 | 200 | 200 |
| Mf-1 | 1.6 | 100 | 1.6 | 25 | 50 |
| Sa-3 | 1.6 | 100 | 1.6 | 25 | 100 |
| Bs-1 | 0.78 | 200 | 6.3 | 200 | 200 |
| Se-3 | 1.6 | 200 | 12.5 | 200 | 200 |
| Sa-4 | 1.6 | 200 | 6.3 | 200 | 200 |
| Pa-2 | - | 100 | 1.6 | 25 | 100 |
| Bf-1 | - | 50 | 12.5 | 100 | 100 |
| Fn-1 | - | 100 | 1.6 | 25 | 50 |
| Pg-1 | - | 50 | 1.6 | 25 | 50 |
| Pt-1 | - | 100 | 12.5 | 100 | 100 |
| Aa-1 | - | 100 | 12.5 | 25 | 200 |
| Av-1 | - | 100 | 3.1 | 25 | 100 |

| | | | | | |
|---|---|---|---|---|---|
| * Each numeric represents unit ppm | | | | | |

**Table 2**

| Test bacteria (Code list of test bacteria used in Examples) | | |
|---|---|---|
| Skin-related bacteria | | |
| Code | Bacteria | Note |
| Se-1 | Staphyrococcus epidermidis JCM 2414 | Resident skin bacteria |
| Se-2 | Staphyrococcus epidermidis var. h-6 | Resident skin bacteria |
| Se-3 | Staphyrococcus epidermidis ATCC 12228 | Resident skin bacteria |
| Cx-2 | Corynebacterium xerosis JCM 1324 | Armpit odor bacteria |
| Mf-1 | Malassezia furfur IFO 0656 | Dandruff-causative bacteria (yeast) |
| Sa-3 | Staphyrococcus aureus 209P IAM 12082 | Staphylococcus aureus |
| Sa-4 | Staphyrococcus aureus ATCC 6538 | Staphylococcus aureus |
| | | |

| Standard bacteria | | |
|---|---|---|
| Code | Bacteria | Note |
| Bs-1 | Bacillus subtilis PCI 219 IFO 3134 | Putrefactive bacteria |
| | | |

| Anaerobic bacteria | | |
|---|---|---|
| Code | Bacteria | Note |
| Pa-2 | Propionibacterium acnes JCM 6473 | Acne bacteria |
| Bf-1 | Bacteroides fragilis GM 7000 JCM 5560 | Abscess bacteria |
| Fn-1 | Fusobacterium nucleatum JCM 6328 | Periodontal bacteria |
| Pt-1 | Prevotella intermedia JCM 6322 | Periodontal bacteria |
| Pg-1 | Porphyromonas gingivalis JCM 8525 | Periodontal bacteria |
| Aa-1 | Actinomyces naeslundii JCM 8350 | Cariogenic bacteria |
| Av-1 | Actinomyces viscosus JCM 8352 | Cariogenic bacteria |

According to the above results, the antibacterial composition (A) as one aspect of the invention was found to show higher antibacterial effects against all of the above bacteria than the conventional antibacterial agent BPHB. Although triclosan has the strongest antibacterial effect, it is refrained from using as it is a halogen-containing compound and thus is concerned about effects to the environment and human body. Antibacterial composition(F) and antibacterial composition(G) comprising component (a) and component (b) respectively also showed some antibacterial effect. However, antibacterial composition(F) and antibacterial composition (G) obviously had narrower antibacterial spectra than that of antibacterial composition(A) produced by mixing component (a) and component (b), and even when antibacterial effect was seen, it was never stronger than that of antibacterial composition(A). Accordingly, it is hard to say that antibacterial composition(F) and antibacterial composition(G) have adequate antibacterial effects. As can be appreciated from the results for antibacterial composition(A), an antibacterial composition with an effect at a practical level can be obtained only after mixing component (a) and component (b).

### [EXAMPLE: 4]

### Comparison of antibacterial activities at different mixture ratios

Minimum inhibitory concentration was determined by Agar Dilution Test described in Example 3 to confirm the difference of antibacterial activities at different mixture ratios. Results are as follows.

**Table 3**

| Antibacterial composition | (A) | (B) | (C) | (D) | (E) | (F) | (G) |
|---|---|---|---|---|---|---|---|
| Pt-1 | 12.5 | 25 | 25 | 50 | 50 | 100 | 100 |
| Bs-1 | 6.3 | 12.5 | 12.5 | 25 | 50 | 200 | 200 |
| Sa-4 | 6.3 | 6.3 | 12.5 | 25 | 50 | 200 | 200 |
| Bf-1 | 12.5 | 12.5 | 12.5 | 50 | 25 | 100 | 100 |

| | | | | | | | |
|---|---|---|---|---|---|---|---|
| * Each numeric represents unit ppm | | | | | | | |

According to the above results, the antibacterial composition of the invention shows the strongest antibacterial activity when component (a) and component (b) are at a ratio of 1:8 to 8:1. An antibacterial composition with component (a) and component (b) at a ratio of 1:30 and 30:1 also showed stronger antibacterial effect than the antibacterial composition with component (a) or component (b) alone. A minimum inhibitory concentration of, for example, 100 ppm to 50 ppm means that the amount of the antibacterial composition added to obtain a certain antibacterial effect can be reduced to half, which makes a great difference in economical aspect.

### [EXAMPLE: 5]

### Determination of Minimum Inhibitory Concentration (MIC) using heat-resistant acidophilic bacteria

Spores of *Alicyclobacillus Acidocaldarius* (NCIMB) and *Alicyclobacillus Acidoterrestris* (NCIMB) were diluted with buffered saline to 1-2 million/100 *µ*l, heated at 80°C for 10 minutes, and added for 100 *µ*l each per 5 ml of AAM *(Alicyclobacillus Acidoterrestris* medium) liquid medium. To the test media prepared as above, antibacterial composition(A) obtained in Example 1 was added according to the following steps. Twenty mg/ml, 10 mg/ml, 5 mg/ml and 2.5 mg/ml antibacterial composition(A) in grapefruit flavors were added to respective media (0.1%) (final concentration of antibacterial composition(A): 20 ppm, 10 ppm, 5 ppm and 25 ppm). As controls, a medium with 0.1% grapefruit flavor and a blank medium were prepared to confirm that the bacteria grow without a problem. The thus-prepared media were cultured at 50°C for 10 days, bacteria growth was determined based on the turbidity of the media associated with proliferation of bacteria, thereby checking growth control effects. For each test bacterium and each sample, five media were subjected to the experiment, and a concentration at which bacteria in every media were inhibited was determined as the minimum inhibitory concentration (MIC).

For comparison, sucrose fatty acid ester was also determined. Results were as follows.

**Table 4**

| Sample | *Alicyclobacillus Acidocaldarius* | *Alicyclobacillus Acidoterrestris* |
|---|---|---|
| Antibacterial composition (A) | 10ppm | 10ppm |
| Sucrose fatty acid ester | 20ppm | 20ppm |

According to the above results, an antibacterial composition of the invention was found to have improved antibacterial effect against heat-resistant acidophilic bacteria than sucrose fatty acid ester does.

The antibacterial composition (A) prepared in Example 1 was used to prepare cosmetic products, food products and oral products having the following compositions.

### [EXAMPLE: 6]

| Tooth paste (unit: % by weight) | |
|---|---|
| Dicalcium phosphate | 10.0 |
| Lauryl sodium sulfate | 2.0 |
| Sodium carboxymethylcellulose | 0.5 |
| Saccharin sodium | 0.02 |
| Mint flavor | 1.0 |
| Antibacterial composition(A) | 0.05% |
| Glycerine | Optimum quantity |
| | 100.0 |

### [EXAMPLE: 7]

| Mouth wash (unit: % by weight) | |
|---|---|
| Ethyl alcohol | 10.0 |
| Polyoxyethylene hydrogenated castor oil | 2.0 |
| Mint flavor | 0.5 |
| Saccharin sodium | 0.02 |
| Glycerine | 10.0 |
| Pigment | Optimum quantity |
| Antibacterial composition(C) | 0.25 |
| Purified water | Optimum quantity |
| | 100.0 |

### [EXAMPLE: 8]

| Candy (unit: % by weight) | |
|---|---|
| Powdered sugar | 50.0 |
| Starch syrup | 33.0 |
| Citric acid | 1.0 |
| Antibacterial composition (C) | 0.25 |
| Purified water | Optimum quantity |
| | 100.0 |

### [EXAMPLE: 9]

| Chewing gum (unit % by weight) | |
|---|---|
| Gum substrate | 21.0 |
| Powdered sugar | 63.9 |
| Com starch | 12.5 |
| Lemon flavor | 1.0 |
| Acidifying agent | 0.6 |
| Antibacterial composition (A) | 1.0 |
| | 100.0 |

### [EXAMPLE: 10]

| Lozenge (unit: % by weight) | |
|---|---|
| Starch | 98.45 |
| Powdered mint flavor | 0.8 |
| Sucrose fatty acid ester | 0.5 |
| Antibacterial composition (B) | 0.25 |
| | 100.0 |

### [EXAMPLE: 11]

| Sanitizer (unit: % by weight) | |
|---|---|
| Ethanol | 20.0 |
| Antibacterial composition (C) | 5.0 |
| Purified water | Optimum quantity |
| | 100.0 |

### [EXAMPLE: 12]

| Powdered detergent (unit: g) | |
|---|---|
| Lauryl, stearyl sodium sulfate | 15.0 |
| Sodium carbonate | 15.0 |
| Sodium metasilicate | 13.0 |
| Sodium citrate | 15.0 |
| Carboxymethylcellulose | 2.0 |
| Sodium sulfate | 38.0 |
| Musk tree perfume | 1.0 |
| Antibacterial composition (A) | 1.0 |
| | 100.0 |

### [EXAMPLE: 13]

| Anhidrotic (unit: g) | |
|---|---|
| PEG-7 glyceryl cocoate | 2.0 |
| Hydrogenated oil | 5.0 |
| Myristyl myristate | 15.0 |
| Cyclomethycone | 33.5 |
| Stearyl alcohol | 20.0 |
| Stearyl isononenoate | 3.0 |
| Aluminum chrolohydrate | 20.0 |
| Fragrance ingredient for anhidrotic | 0.5 |
| Antibacterial composition(C) | 1.0 |
| | 100.0 |

### [EXAMPLE: 14]

| Emollient cream (unit: g) | |
|---|---|
| Cetyl alcohol | 5.0 |
| Stearic acid | 3.0 |
| Vaseline | 5.0 |
| Squalane | 10.0 |
| Glycerol tri2-ethylhexanoate ester | 7.0 |
| Dipropylene glycol | 5.0 |
| Glycerine | 5.0 |
| Propylene glycol monostearate ester | 3.0 |
| POE(20) cetyl alcohol ether | 3.0 |
| Triethanolamine | 1.0 |
| Paraben | 0.3 |
| Fragrance ingredient for cream | 1.0 |
| Antibacterial composition(C) | 1.0 |
| Purified water | Optimum quantity |
| | 100.0 |

### [EXAMPLE: 15]

| Shampoo (unit: g) | |
|---|---|
| Sodium laureth sulfate | 40.0 |
| Sodium cocoamphoacetate | 10.0 |
| Cocamide DEA | 2.0 |
| Butylene glycol | 2.0 |
| Citric acid | 035 |
| Sodium chloride | 0.1 |
| Paraben | 0.3 |
| Tetrasodium EDTA | 0.1 |
| Fragrance ingredient for shampoo | 0.5 |
| Antibacterial composition(A) | 1.0 |
| Purified water | Optimum quantity |
| | 100.0 |

### [EXAMPLE: 16]

| Juice-containing beverage (unit: g) | |
|---|---|
| Fructose/glucose liquid sugar | 107.0 |
| Citric acid | 1.0 |
| Sodium citrate | 0.3 |
| Concentrated orange juice | 51.8 |
| Water-soluble orange flavor | 1.0 |
| Antibacterial composition(B) | 0.1 |
| Water | Optimum quantity |
| | 1000.0 |

### [EXAMPLE: 17]

| Sports drink (unit: g) | |
|---|---|
| Sugar | 31.0 |
| Glucose | 15.7 |
| Citric acid | 1.0 |
| Calcium lactate | 0.679 |
| Sodium citrate | 03 |
| Sodium chloride | 0.28 |
| Potassium chloride | 0.22 |
| Vitamin C | 0.864 |
| Sodium L-glutamate | 0.03 |
| Niacin | 0.013 |
| Calcium pantothenate | 0.007 |
| Vitamin B6 | 0.0022 |
| Vitamin B12 | 0.000006 |
| Lemon flavor | 1.0 |
| Antibacterial composition (B) | 0.1 |
| Purified water | Optimum quantity |
| | 1000.0 |

### [EXAMPLE: 18]

| Coffee milk beverage (unit: g) | |
|---|---|
| Regular coffee | 50.0 |
| Caster sugar | 50.0 |
| Milk | 150.0 |
| Emulsifying agent (fatty acid ester) | 0.5 |
| Coffee flavor | 1.0 |
| Milk flavor | 0.8 |
| Antibacterial composition (A) | 0.1 |
| Purified water | Optimum quantity |
| | 1000.0 |

### [EXAMPLE: 19]

| Carbonated drink (unit: g) | |
|---|---|
| Fructose/glucose liquid sugar | 127.0 |
| Citric acid | 1.24 |
| Purified water | 200.0 |
| Lemon flavor | 0.12 |
| Antibacterial composition (B) | 0.05 |
| Carbonated water | Optimum quantity |
| | 1000.0 |

### [EXAMPLE: 20]

| Juice-containing jelly (unit: g) | |
|---|---|
| Apple juice | 6.0 |
| Starch syrup | 3.5 |
| Caster sugar | 13.0 |
| Malic acid | 0.21 |
| Gelling agent | 0.9 |
| Sodium citrate | 0.05 |
| Caramel pigment | 0.08 |
| Apple flavor | 0.2 |
| Antibacterial composition(C) | 0.01 |
| Purified water | Optimum quantity |
| | 100.0 |

### [EXAMPLE: 21]

| Lemon tea (unit: g) | |
|---|---|
| Tea leaf extract (brix 1.0) | 200.0g |
| Caster sugar | 60.0 |
| Lemon concentrated juice | 1.56 |
| Vitamin C | 0.1 |
| Antibacterial composition (A) | 0.05 |
| Purified water | Optimum quantity |
| | 1000.0 |

No turbidity or sediment was visually found in the acidic beverages prepared above (Examples 16,17,19 and 21).

The antibacterial composition obtained according to the present invention has an improved antibacterial effect. Specifically, it exerts improved antibacterial activity against various bacteria including oral-related bacteria such as cariogenic bacteria and periodontal bacteria, food poisoning bacteria and putrefactive bacteria concerned in the food industry, and acne bacteria, dandruff-causative bacteria and resident skin bacteria concerned in the cosmetic industry. Thus, the present antibacterial composition can be applied to wide variety of products such as food products, cosmetic products (fragrance products, skin-care cosmetics, hair-care cosmetics, toiletry products, bath additives, body-care products, detergents, softeners, aromatic deodorants), oral products and pharmaceutical products. Furthermore, the antibacterial composition of the present invention may be used with other antibacterial agents or substances having antibacterial activities and thus synergistic antibacterial effect thereof can be expected. By using the antibacterial composition of the present invention in the above-mentioned products, improved antibacterial effect and preservation stability can be provided.

## Claims

1. An antibacterial composition comprising 9-(3',7'-dimethyl-2',6'-octadienyl)-4-hydroxy-7H-furo[3,2-γ][1]-benzopyran-7-one and 8-(3',7'*-*dimethyl-2',6'-octadienyl)-5-hydroxy-7-methoxy-2H-1-benzopyran-2-one.

2. An antibacterial composition according to Claim 1, comprising 9-(3',7'-dimethyl-2',6'-octadienyl)-4-hydroxy-7H-furo[3,2-γ][1]benzopyran-7-one and 8-(3',7'-dimethyl-2',6'-octadienyl)-5-hydroxy-7-methoxy -2H-1-benzopyran-2-one at a weight ratio of 1:30 to 30:1.

3. A food product comprising the antibacterial composition of Claim 1 or 2.

4. An oral product comprising the antibacterial composition of Claim 1 or 2.

5. A cosmetic product comprising the antibacterial composition of Claim 1 or 2.

6. A pharmaceutical product comprising the antibacterial composition of Claim 1 or 2

7. Use of a composition comprising 9-(3',7'-dimethyl-2',6'-octadienyl)-4-hydroxy-7H-furo[3,2-γ][1]benzopyran-7-one and 8-(3',7'-dimethyl-2',6'-octadienyl)-5-hydroxy-7-methoxy-2H-1-benzopyran-2-one as an antiseptic or disinfectant agent.

## Patentansprüche

1. Antibakterielle Zusammensetzung, umfassend 9-(3',7'-Dimethyl-2',6'-octadienyl)-4-hydroxy-7H-furo[3,2-γ][1]benzopyran-7-on und 8-(3',7'-Dimethyl-2',6'-octadienyl)-5-hydroxy-7-methoxy-2H-1-benzopyran-2-on.

2. Antibakterielle Zusammensetzung nach Anspruch 1, umfassend 9-(3',7'-Dimethyl-2',6'-octadienyl)-4-hydroxy-7H-furo[3,2-γ][1]benzopyran-7-on und 8-(3',7'-Dimethyl-2',6'-octadienyl)-5-hydroxy-7-methoxy-2H-1-benzopyran-2-on bei einem Gewichtsverhältnis von 1 : 30 bis 30 : 1.

3. Nahrungsmittelprodukt, umfassend die antibakterielle Zusammensetzung nach Anspruch 1 oder 2.

4. Orales Produkt, umfassend die antibakterielle Zusammensetzung nach Anspruch 1 oder 2.

5. Kosmetisches Produkt, umfassend die antibakterielle Zusammensetzung nach Anspruch 1 oder 2.

6. Pharmazeutisches Produkt, umfassend die antibakterielle Zusammensetzung nach Anspruch 1 oder 2.

7. Verwendung einer Zusammensetzung, umfassend 9-(3',7'-Dimethyl-2',6'-octadienyl)-4-hydroxy-7H-furo[3,2-γ][1]benzopyran-7-on und 8-(3',7'-Dimethyl-2',6'-octadienyl)-5-hydroxy-7-methoxy-2H-1-benzopyran-2-on als ein antiseptisches oder desinfizierendes Mittel.

## Revendications

1. Composition antibactérienne comprenant de la 9-(3',7'-diméthyl-octa-2',6'-diényl)-4-hydroxy-7H-furo[3,2-y][1]benzopyran-7-one et de la 8-(3',7'-diméthyl-octa-2',6'-diényl)-5-hydroxy-7-méthoxy-2H-1-benzopyran-2-one.

2. Composition antibactérienne conforme à la revendication 1, comprenant de la 9-(3',7'-diméthyl-octa-2',6'-diényl)-4-hydroxy-7H-furo[3,2-γ]-[1]benzopyran-7-one et de la 8-(3',7'-diméthyl-octa-2',6'-diényl)-5-hydroxy-7-méthoxy-2H-1-benzopyran-2-one en un rapport pondéral de 1/30 à 30/1.

3. Produit alimentaire comprenant une composition antibactérienne conforme à la revendication 1 ou 2.

4. Produit buccal comprenant une composition antibactérienne conforme à la revendication 1 ou 2.

5. Produit cosmétique comprenant une composition antibactérienne conforme à la revendication 1 ou 2.

6. Produit pharmaceutique comprenant une composition antibactérienne conforme à la revendication 1 ou 2.

7. Emploi, à titre d'agent désinfectant ou antiseptique, d'une composition comprenant de la 9-(3',7'-diméthyl-octa-2',6'-diényl)-4-hydroxy-7H-furo[3,2-γ][1]benzopyran-7-one et de la 8-(3',7'-diméthyl-octa-2',6'-diényl)-5-hydroxy-7-méthoxy-2H-1-benzopyran-2-one.
